# EUROPEAN PATENT SPECIFICATION

(11) **EP 1 531 873 B1**
(45) Date of publication and mention of the grant of the patent: **21.09.2011**
(21) Application number: 03749269.1
(22) Date of filing: 29.08.2003
(51) Int. Cl.: A61K 51/00, A61K 51/06, A61K 51/10

(54) **AGENT DELIVERY PARTICLE**
PARTIKEL ZUR WIRKSTOFFABGABE
PARTICULE D'ADMINISTRATION D'AGENT

(30) Priority: 30.08.2002 US 232265; 08.07.2003 US 615276
(43) Date of publication of application: 25.05.2005
(73) Proprietor: Boston Scientific Limited, St. Michael, Barbados, West Indies (BB)
(72) Inventor: DIMATTEO, Kristian, Waltham, MA 02451 (US); CASEY, Thomas, V., II, Grafton, MA 01519 (US); RIOUX, Robert, F., Ashland, MA 01721 (US); KEENAN, Steve, Framingham, MA 01701 (US); LANPHERE, Janel, Newton, MA 02461 (US)
(74) Representative: Lang, Johannes
(86) International application number: PCT/US2003/027228
(87) International publication number: WO 2004/019999

(56) References cited:
- WO-A-02/34300
- WO-A-99/51278
- DE-A- 10 026 620
- US-B1- 6 214 384
- DATABASE WPI Section Ch, Week 199716 Derwent Publications Ltd., London, GB; Class A96, AN 1997-168090 XP002269188 -& ES 2 096 521 A (UNIV LAGUNA), 1 March 1997 (1997-03-01)
- DATABASE WPI Section Ch, Week 199735 Derwent Publications Ltd., London, GB; Class B06, AN 1997-381275 XP002269189 -& JP 09 165328 A (HARADA N), 24 June 1997 (1997-06-24)

## Description

### TECHNICAL FIELD

This invention relates to an agent delivery particle.

### BACKGROUND

Agents, such as therapeutic agents, can be delivered systemically, for example, by injection through the vascular system or oral ingestion, or they can be applied directly to a site where treatment is desired. In the case of delivery of a therapeutic agent, it is often desirable that the therapeutic agent be delivered at desired dosages for an extended period of time.

### SUMMARY

The invention relates to agent delivery particles as defined in claim 1.

In one aspect, the invention features a composition that includes a substantially spherical polymer particle having a diameter of about 1200 microns or less and containing an agent which includes a radioactive species.

In another aspect, the invention features the use of the composition for preparing a medicament. The composition includes a substantially spherical polymer particle having a diameter of about 1200 microns or less containing an agent which includes a radioactive species.

In a further aspect, the invention features a method of making a composition that includes a substantially spherical polymer particle having a diameter of about 1200 microns or less and containing an agent which includes a radioactive species.

In an additional aspect, the invention features a method of making a composition that includes a substantially spherical polymer particle having a diameter of about 1200 microns or less and containing an agent which includes a radioactive species.

Embodiments can include one or more of the following features.

The particle can have an interior and a surface region, with a density of large pores in the interior that is greater than the density of large pores at the surface region of the particle.

The agent can be a therapeutic agent.

The radioactive species can be a radioactive molecule.

The radioactive species can be a radioisotope. Examples of radioisotopes include yttrium (⁹⁰Y), lutetium (¹⁷⁷Lu) , actinium (²²⁵Ac), praseodymium, astatine (²¹¹At), rhenium (¹⁸⁶Re), bismuth (²¹²Bi or ²¹³Bi), holmium (¹⁶⁶Ho), samarium (¹⁵³Sm), iridium (¹⁹²Ir), rhodium (¹⁰⁵Rh), iodine (¹³¹I or ¹²⁵I), indium (¹¹¹In), technetium (⁹⁹Tc), phosphorus (³²P), sulfur (³⁵S), carbon (¹⁴C), tritium (³H), chromium (⁵¹Cr), chlorine (³¹Cl), cobalt (⁵⁷Co or ⁵⁸Co), iron (⁵⁹Fe), selenium (⁷⁵Se) and gallium (⁶⁷Ga).

The radioisotope can be bound to an antibody. Examples of antibodies include RS7, hRS7, MOv18, MN-14 IgG, CC49, COL-1, NP-4 F(ab') 2 anti-CEA, anti-PSMA, ChL6, m-170, and antibodies to CD20, antibodies to CD74, and CD52 antigens.

The antibody can be a monoclonal antibody. Examples of monoclonal antibodies include mAB A33, m-170, antibodies to CD20, antibodies to CD74, and antibodies to CD52 antigens.

Examples of polymers include polyvinyl alcohol, polycaprolactone, polylactic acid, and poly(lactic-co-glycolic) acid.

The agent can be included in the interior of the particle.

The agent can be on the surface region of the particle.

The composition can be used to treat a cancer condition. Examples of cancer conditions include ovarian cancer, colorectal cancer, thyroid cancer, gastrointestinal cancer, breast cancer, prostate cancer and lung cancer.

The antibody can bind to antigens at a treatment site of the subject.

The radioactive species can be released at the treatment site.

The composition can be delivered by percutaneous injection.

The composition can be delivered by a catheter.

Embodiments can include one or more of the following advantages.

In some embodiments, a sustained, controlled-dosage release of an agent (e.g., a therapeutic agent) can be effected by a substantially spherical agent-containing particle that includes a reservoir region in its interior and a metering region surrounding the reservoir by, for example, coating the surface of the particle with agent.

In certain embodiments, a burst release of an agent (e.g., a therapeutic agent) can be effected by a substantially spherical agent-containing particle that includes a reservoir region in its interior and a metering region surrounding the reservoir region by, for example, loading the interior of the particle with agent.

In some embodiments, a combination sustained, controlled-dosage release and burst release of agent (e.g., therapeutic agent) can be obtained with a substantially spherical agent-containing particle that includes a reservoir region in its interior and a metering region surrounding the reservoir by, for example, coating the surface of the particle with agent and loading the interior of the particle with agent. In certain embodiments, the agent coated on the surface can first be released in a controlled manner, followed by a burst release of the agent loaded in the interior of the particle.

In embodiments, loading the agent onto and/or into the particle can target (e.g., physically target) the agent to a desired site (e.g., a site having a condition to be treated, such as a site having a cancer condition). This can allow for a more efficient use of agent. For example, targeted delivery can permit a lower dosage of agent to be used. This can reduce side effects due to the agent.

In certain embodiments, the agent can be bound to an antibody (e.g., a radioisotope bound to an antibody) which can further enhance the targeting of a desired treatment site (e.g., via chemical recognition of the antibody by antigens at the treatment site).

Other aspects, features, and advantages follow.

### DESCRIPTION OF DRAWINGS

FIG. 1 is a schematic illustrating administration of agent delivery particles.
FIG. 2 is a cross-sectional schematic illustrating release of agent from a particle.
FIG. 3A is a light micrograph of a collection of particles; FIG. 3B is a scanning election microscope (SEM) photograph of a particle surface; and FIGS. 3C - 3E are SEM photographs of cross-sections of particles.
FIG. 4A is a schematic of the manufacture of a particle and FIG. 4B is an enlarged schematic of region A in FIG. 4A.
FIG. 5 is a photograph of a gel-stabilized drop.
FIG. 6 is a graph of particles in uniformity.

### DETAILED DESCRIPTION

### Structure

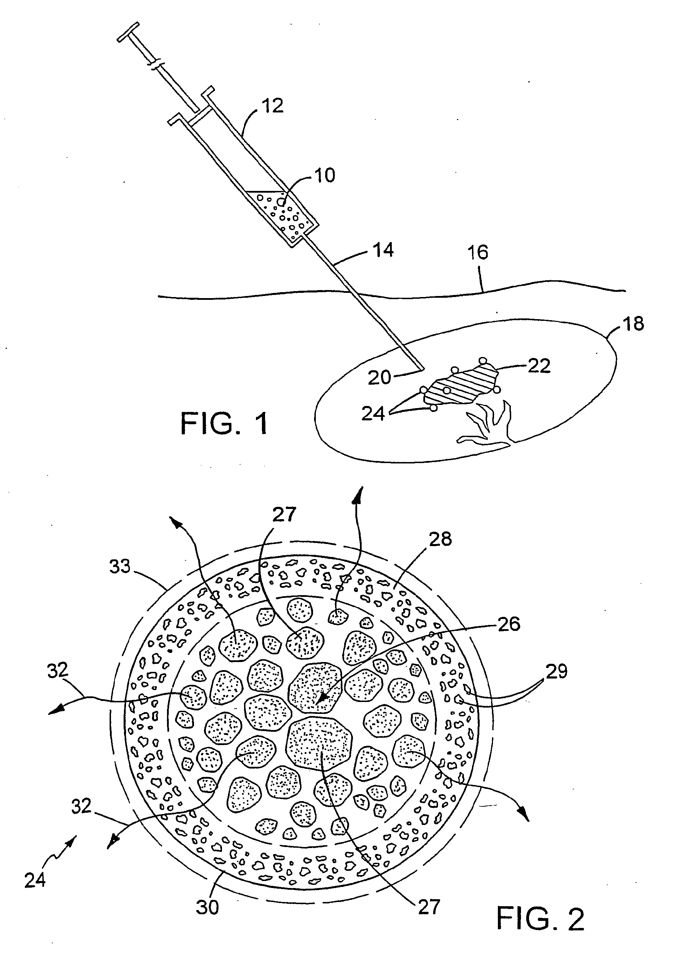

Referring to Fig. 1, a composition 10 is injected using a syringe 12 with a needle 14 that is used to puncture the skin 16 and extend into an organ 18. The tip 20 of needle 14 is disposed within the tissue mass of the organ near and/or within a tumorous malignancy 22. The composition 10 includes a carrier fluid which carries agent delivery particles 24. The particles can be positioned about the lesion 22. In some embodiments, syringe 12 can be made of glass. Examples of commercially available syringes are available from Becton-Dickinson (Franklin Lakes, NJ). In certain embodiments, such as when the agent is a radioactive species, the syringe can be equipped with a shield to protect the user. Examples of commercially available syringe shields include the Pro-Tec series of syringe shields and the high density lead glass syringe shields (Gammasonics, NSW 2046, Australia).

In certain embodiments, the particles can be delivered through the vasculature, e.g., by a catheter inserted into the hepatic artery. In some embodiments, the particles can be delivered using a microcatheter. Examples of commercially available microcatheters include the Renegade^{®} Hi-FLO microcatheter systems (Boston Scientific Corporation, Maple Grove, MN).

Composition 10 can be delivered to various sites in the body, including, for example, sites having cancerous lesions, such as the breast, prostate, lung, thyroid, or ovaries.

Referring particularly to FIG. 2, the particles are substantially spherical and include an interior reservoir region 26 which is characterized by relatively large pores 27 and a metering region 28 which is characterized by relatively small pores 29. The large pores 27 in the reservoir region hold a supply of an agent, such as a radioactive species-containing agent (e.g., a tumor-toxic agent), which diffuses through interpore passageways into the metering region and is released from the surface 30 of the particle (arrows 32) to adjacent tissue. The porous structure of the particle is believed to create an agent concentration gradient from relatively high therapeutic concentration in the reservoir region to lower concentrations in the metering region. The relative size of the pores in the regions and the relative thickness of the metering region control the rate of elution of agent (e.g., therapeutic agent) from the particle. The substantially spherical shape of the particle contributes to symmetric elution in all directions. In addition, the relatively uniform thickness of the metering region surrounding the reservoir region enhances uniformity of elution dosage.

The particles are substantially formed of a highly water-insoluble, high molecular weight polymer. As will be discussed further below, a preferred polymer is high molecular weight polyvinyl alcohol (PVA) that has been acetalized. Preferably, the embolic particles are substantially pure intrachain 1,3 acetalized PVA and substantially free of animal derived residue such as collagen. In embodiments, the particles include a minor amount, e.g. less than about 0.2 weight %, of alginate or another polysaccharide or gelling material. The particles can also be formed of polycaprolactone (PCL), polylactic acid (PLA) or poly(lactic-co-glycolic) acid (PLGA). The particle may also include an optional coating 33. The coating erodes in the body, e.g. on contact with body fluid, as will be discussed below.

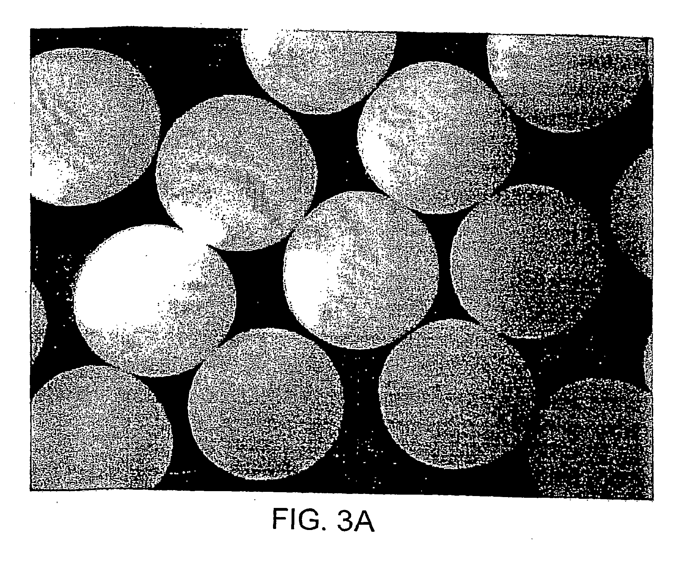

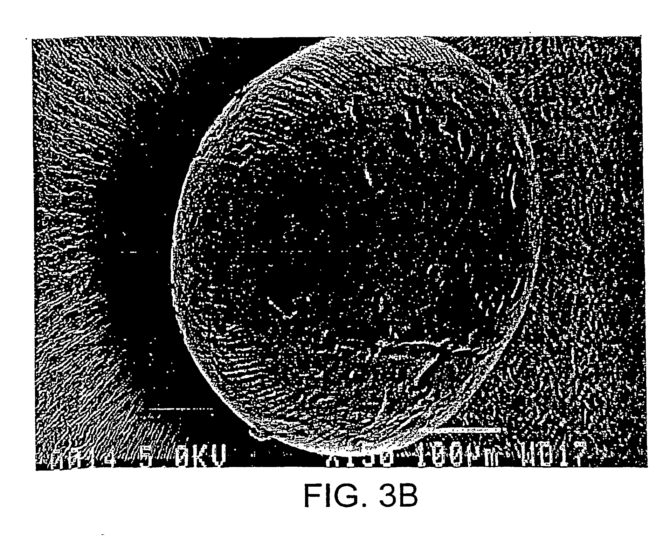

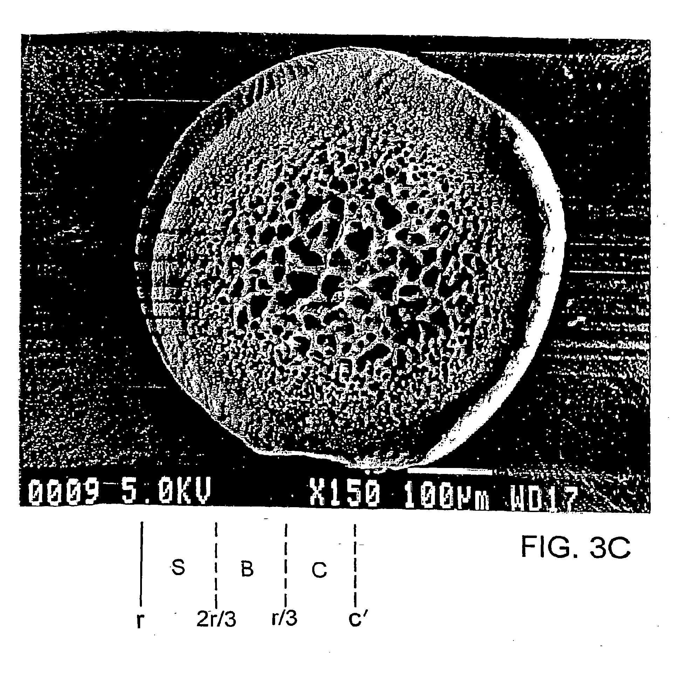

Referring to FIG. 3A, the particles have a substantially uniform shape and size. Referring to FIG. 3B, each particle has a well-defined outer spherical surface including relatively small, randomly located pores. Referring to FIGS. 3C - 3E, SEM images of cross-sections through the particle, the body defines pores that provide metering of agent (e.g., therapeutic agent) release, as well as compressibility and other properties.

In embodiments, the small pore region near the periphery of the embolic particle is relatively stiff and incompressible, which enhances resistance to shear forces and abrasion. In addition, the variable pore size profile produces a symmetric compressibility and, it is believed, a compressibility profile such that the particles are relatively easily compressed from a maximum, at rest diameter to a smaller, compressed first diameter but compression to even smaller diameter requires substantially greater force. A variable compressibility profile is believed to be due to the presence of a relatively weak, collapsible inter-pore wall structure in the center region where the pores are large, and a stiffer inter-pore wall structure near the surface of the particle, where the pores are more numerous and relatively small. The variable pore size profile also is believed to enhance elastic recovery after compression. The pore structure also influences the density of the embolic particles and the rate of agent (e.g., therapeutic agent) and body fluid uptake.

The particles can be delivered through a syringe, a catheter or a microcatheter as discussed earlier. The size of the lumen of the syringe or the catheter can be larger than the particle diameter to reduce compression of the particles during delivery, which can eject agent (e.g., therapeutic agent) from the particle prematurely. While compression can result in release of agent, the metering region can retard substantial release under low compression force. In embodiments, the particles are compressed during delivery in order to use a delivery device that has a small diameter to reduce patient trauma or more accurately position the particles about a lesion. The carrier fluid in which the particles are suspended can include agent so that upon recovery to normal diameter, the agent is drawn into the pores of the particle. For example, the particles can be delivered through a catheter having a lumen area that is smaller, e.g. 50% smaller or less, than the uncompressed cross-sectional area of the particles. The compression force is provided indirectly by increasing the pressure applied to the carrier fluid by pressing the syringe plunger. The particles are relatively easily compressed to diameters sufficient for delivery into the body. The robust, rigid surface region resists abrasion when the embolic particles contact hard surfaces such as syringe surfaces, hard plastic or metal stopcock surfaces, and the catheter lumen wall (e.g. Teflon^{®}) during delivery. Once in the body, the particles recover to original diameter for efficient transport in the carrier and body fluid stream. At the point of occlusion, the particles can again compress as they aggregate in an occlusion region. The particles form a dense occluding mass. The compression in the body is limited and the number of embolic particles needed to occlude a given diameter may be reduced. The particles can also be delivered directly into a tissue mass where re-expansion to a larger diameter firmly lodges the particle into the tissue.

In general, the particles have a diameter of about one centimeter or less (e.g., about five mm or less, about one mm or less). In certain embodiments, the particles have a diameter of at least about 10 microns (e.g., at least about 100 microns, at least about 200 microns, at least about 400 microns) and at most about 1200 microns. For example, the particles can have a diameter from about 100 microns to about 700 microns, from about 500 microns to about 700 microns, from about 100 microns to about 500 microns, from about 300 microns to about 500 microns, from about 700 microns to about 900 microns, from about 900 microns to about 1200 microns. In embodiments, the pores are at most about 50 microns (e.g., at most about 35 microns) and at least about 0.01 micron. The particles have a mean diameter in approximately the middle of the range and a variance of at most about 20% (e.g., at most about 15%, at most about 10%).

Referring specifically to FIG. 3C, the particles can be considered to include a center region, C, from the center c' of the particle to a radius of about r/3; a body region, B, from about r/3 to about 2r/3; and a surface region, S, from 2r/3 to r. The regions can be characterized by the relative size of the pores and the number of pores of given sizes. In embodiments, the center region has a greater number of relatively large pores than the body region and the surface region. The large pores are in the range of about 20 microns or more, e.g., 30 microns or more, or in the range of about 20 to 35 microns. The body region has a greater number of intermediate size pores than the surface region. The intermediate size pores are in the range of about five to 18 microns. In embodiments, the regions may also have different densities, with the density of the surface region being greater than the density of the body region, and the density of the body region being greater than the density of the center region.

The size of the pores in each of the regions can also be characterized by a distribution. In embodiments, the predominant pore size(s) in the center region is greater than the predominant pore size(s) in the body region and the predominant pore size(s) in the body region is greater than the predominant pore size(s) in the surface region. In embodiments, the predominant pore size in the center region is 20 microns or more, e.g. 30 microns or more, or in the range of about 20 to 35 microns. The predominant pore size in the body region is about 18 microns or less, e.g. about 15 microns or less, or in the range of about 18 to two microns. The pores in the surface region are preferably predominantly less than about one micron, e.g. about 0.1 to 0.01 micron.

In embodiments, the predominant pore size in the body region is about 50 to 70% of the pore size in the center region and the pore size in the surface region is about 10% or less, e.g. about 2% of the pore size in the body region. The size of the pores on the outer surface of the particle is predominantly in the range of about one micron or less, e.g. about 0.1 or 0.01 micron. In embodiments, the surface and/or surface region is substantially free of pores having a diameter larger than about 10 microns or larger than about one micron. In embodiments, the predominant pore size is in the region 0.8 or 0.9r to r is about one micron or less, e.g. 0.5 to 0.1 micron or less. The region from the center of the particle to 0.8 or 0.9r has pores of about 10 microns or greater and/or has a predominant pore size of about 2 to 35 microns. In embodiments, the predominant pore size in the region 0.8 or 0.9r to r is about 5% or less, e.g. 1% or 0.3% or less than the predominant pore size in the region from the center to 0.9r. The largest pores in the particles can have a size in the range of 1% or 5% or 10% or more of the particle diameter.

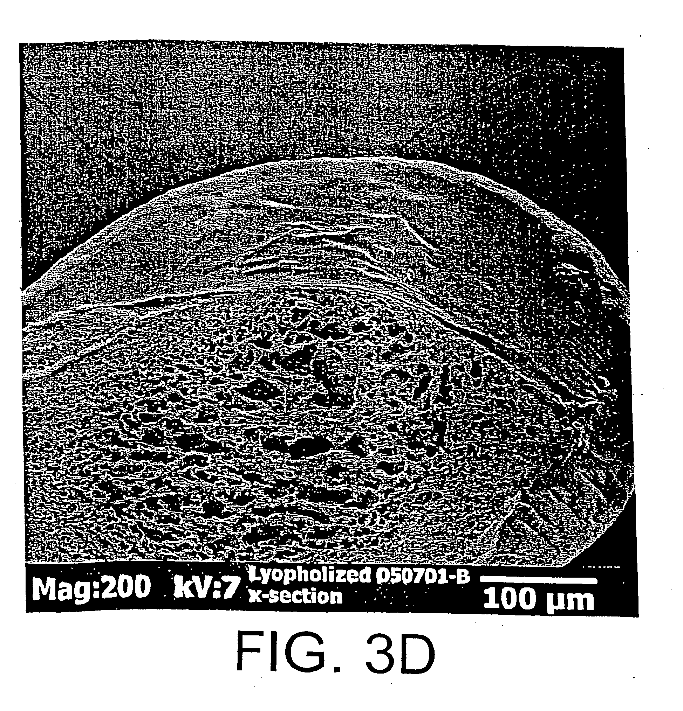

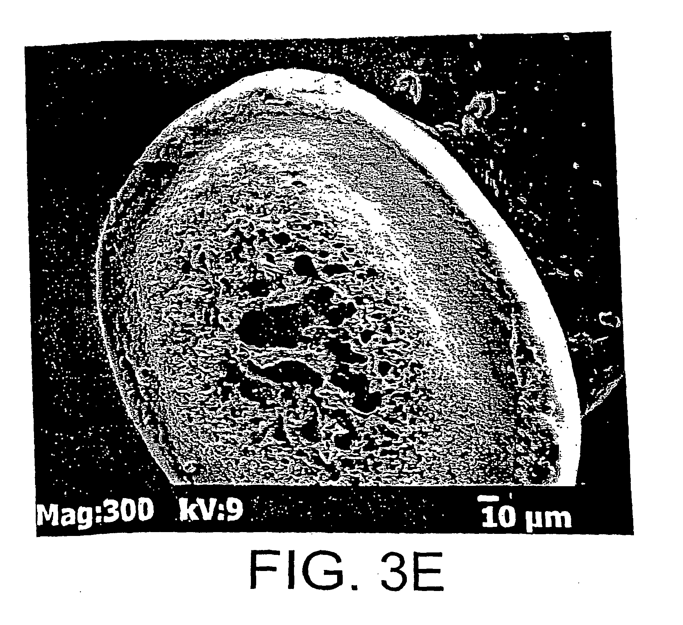

The size of the pores can be measured by viewing a cross-section as in Fig. 3C. For irregularly shaped pores, the maximum visible cross-section is used. The predominant pore size(s) can be found by measuring the size of the visible pores and plotting the number of pores as a function of size. The predominant pore size(s) are the sizes that are about the maximum in the distribution. In Fig. 3C, the SEM was taken on wet particles including absorbed saline, which were frozen in liquid nitrogen and sectioned. (Fig. 3B was taken prior to sectioning.) In Figs. 3D and 3E, the particle was freeze-dried prior to sectioning and SEM analysis.

In general, the density of the particles is such that they are readily suspended in the carrier fluid such as a mixture of saline and contrast solution and remain suspended during delivery. Further, the density of particles is generally such that they can be suspended in the body fluid with which they are in contact, for example, blood. In some embodiments, the density is about 1.1 - 1.4g/cm³. For suspension in a saline-contrast solution, the density is about 1.2 - 1.3g/cm³. The sphericity after compression in a catheter to about 50% or more of their cross-sectional area is about 0.90, 0.95, or greater. In embodiments, the particles can be manually compressed, essentially flattened, while wet to less than 50% of original diameter and then, upon exposure to fluid, regain a sphericity of about 0.9 or more. The carrier fluid can be a pharmaceutically acceptable carrier such as saline or contrast agent or therapeutic agent or a combination of these carriers. The particles or composition can be sterilized.

In certain embodiments, the particles can serve as embolic particles. Such particles are disclosed, for example, in U.S. Patent Application Serial No. 10/215,594, filed August 9, 2002 and entitled "Embolization", and U.S. Patent Application Serial No. 10/109,966, filed March 29, 2002 and entitled "Processes for Manufacturing Polymeric Microspheres".

### Manufacture

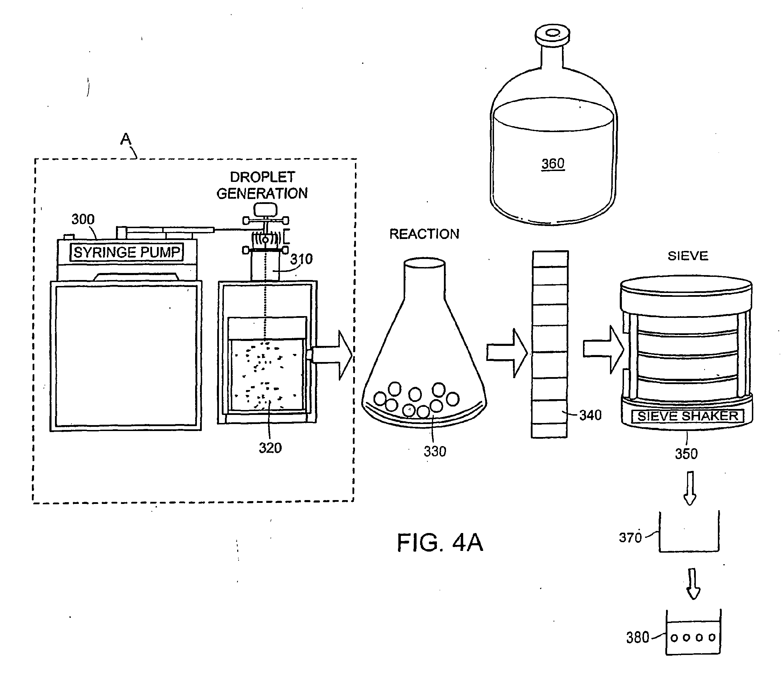

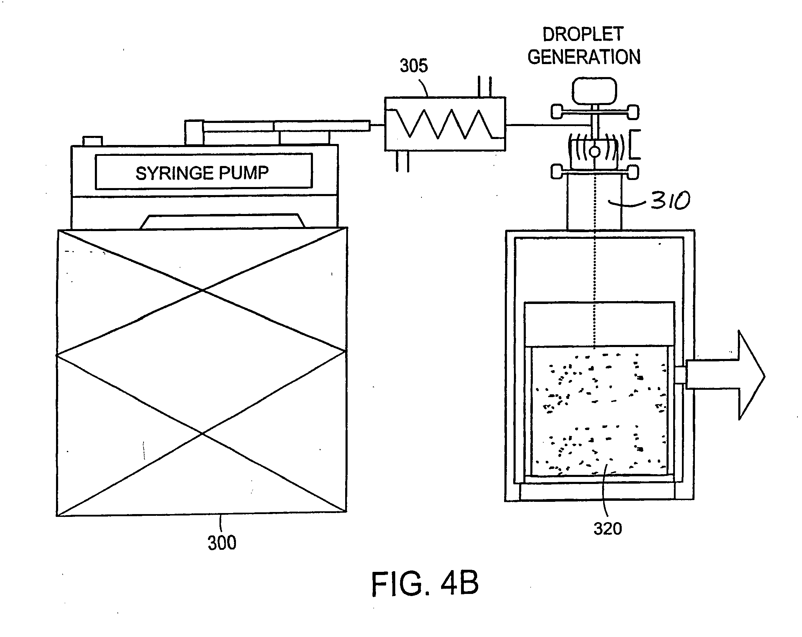

Referring to FIGS. 4A and 4B, a system for producing particles includes a flow controller 300, a drop generator 310, a gelling vessel 320, a reactor vessel 330, a gel dissolution chamber 340, a filter 350, a supply of agent (e.g., therapeutic agent) 360, a particle drying chamber 370, and a particle rehydrator vessel 380. The flow controller 300 delivers polymer solutions to a viscosity controller 305, which heats the solution to reduce viscosity prior to delivery to the drop generator 310. The drop generator 310 forms and directs drops into a gelling vessel 320, where drops are stabilized by gel formation. The gel-stabilized drops are transferred from the gelling vessel 320 to reactor vessel 330 where the polymers in the gel-stabilized drops are reacted, forming precursor particles. The precursor particles are transferred to a gel dissolution chamber 340, where the gel is dissolved. The particles are then filtered in a filter 350 to remove debris, sterilized, and packaged as a composition including the particles. As will be discussed below, the agent can be incorporated into the particles at various stages. In the embodiment illustrated, after filtering, the particles can be dried in a chamber 370, e.g. under vacuum (e.g., by lyophilization) with or without heat application or air dried with or without heat, e.g., at room temperature. The dried particles are then rehydrated in a vessel 380 which includes agent. In the rehydration process, the agent is drawn into the particles through the pore structure. The particles can then be packed in a solution of agent. The particles can be mixed with saline or contrast agent at the time of administration.

A base polymer and a gelling precursor are dissolved in water and mixed. The mixture is introduced to a high pressure pumping apparatus and/or pressurized vessel, such as a syringe pump (e.g., model PHD4400, Harvard Apparatus, Holliston, MA). Examples of base polymers include polyvinyl alcohol, polyacrylic acid, polymethacrylic acid, polyvinyl sulfonate, carboxymethyl cellulose, hydroxyethyl cellulose, substituted cellulose, polyacrylamide, polyethylene glycol, polyamides, polyureas, polyurethanes, polyester, polyethers, polystyrene, polysaccharide, polylactic acid, polyethylene, polymethylmethacrylate, polycaprolactone, polyglycolic acid, and copolymers or mixtures thereof. A preferred polymer is polyvinyl alcohol. The polyvinyl alcohol, in particular, is hydrolyzed in the range of 80 to 99%. The weight average molecular weight of the base polymer can be in the range of 9000 to 186,000, 85,000 to 146,000 or 89,000 to 98,000. Gelling precursors include, for example, alginates, alginate salts, xanthan gums, natural gum, agar, agarose, chitosan, carrageenan, fucoidan, furcellaran, laminaran, hypnea, eucheuma, gum arabic, gum ghatti, gum karaya, gum tragacanth, hyalauronic acid, locust beam gum, arabinogalactan, pectin, amylopectin, other water soluble polysaccharides and other ionically crosslinkable polymers. A particular gelling precursor is sodium alginate. A preferred sodium alginate is high guluronic acid, stem-derived alginate (e.g. about 50 or 60% or more guluronic acid with a low viscosity e.g. about 20 to 80 cps at 20°C) which produces a high tensile, robust gel. High molecular weight PVA is dissolved in water by heating, typically above about 70°C, while alginates can be dissolved at room temperature. The PVA can be dissolved by mixing PVA and alginate together in a vessel which is heated and generally pressurized. In certain embodiments, the vessel is heated to an autoclave temperature about 121°C. Alternatively, the PVA can be disposed in water and heated and the alginate subsequently added at room temperature to avoid exposing the alginate to high temperature. Heat can also be applied by microwave application. For PVA/alginate, the mixture is typically about 7.5 to 8.5%, e.g. about 8% by weight PVA and about 1.5 to 2.5%, e.g. about 2%, by weight alginate.

In certain embodiments, one or more (e.g., all) of the particles are substantially free of ceramic materials, e.g., contain less than one weight % ceramic, contain less than 0.5 weight % ceramic, contain less than 0.1 weight % ceramic.

Referring to FIG. 4B, the viscosity controller 305 is a heat exchanger circulating water at a predetermined temperature about the flow tubing between the pump and drop generator. The mixture of base polymer and gelling precursor flows into the viscosity controller 305, where the mixture is heated so that its viscosity is lowered to a level for efficient formation of very small drops. For a high molecular weight PVA/alginate solution, the temperature of the circulating water is less than about 75°C and more than about 60°C, for example, 65°C which maintains the mixture at a viscosity of 90-200 centipoise. For spherical particles, the viscosity of the drops is maintained so they are captured in the gelling vessel without splintering or cojoining which can create irregular, fibrous particles. In other embodiments, the flow controller and/or the drop generator can be placed in a temperature-controlled chamber, e.g. an oven, or a heat tape wrap, to maintain a desired viscosity.

The drop generator 310 generates substantially spherical drops of predetermined diameter by forcing a stream of the mixture of base polymer and gelling precursor through a nozzle which is subject to a periodic disturbance to break up the jet stream into drops. The jet stream can be broken into drops by vibratory action generated for example, by an electrostatic or piezoelectric element. The drop size is controlled by controlling the flow rate, viscosity, amplitude, and frequency at which the element is driven. Lower flow rates and higher frequencies produce smaller drops. A suitable electrostatic drop generator 310 is available from NISCO Engineering, model NISCO Encapsulation unit VAR D, Zurich, Switzerland. In some embodiments, e.g., to make particles of diameter less than 500 microns, a suitable drop generator from Inotech AG, Dottikon, Switzerland, can be used. In embodiments, the frequency is in the range of about 0.1 to 0.8 kHz. The flow rate through the droplet generator is in the range of about 1 to 12 mL per minute. The drop generator can include charging the drops after formation such that mutual repulsion between drops prevents drop aggregation as they travel from the generator to the gelling vessels. Charging may be achieved by, e.g. an electrostatic charging device such as a charged ring positioned downstream of the nozzle.

Drops of the base polymer and gelling precursor mixture are captured in the gelling vessel 320. The gelling vessel 320 contains a gelling agent which interacts with the gelling precursor to stabilize drops by forming a stable gel. In some embodiments, e.g., to make particles less than 500 microns, the gelling agent can be heated to an appropriate temperature e.g., 30 °C. In some embodiments, e.g., to lower the surface tension, air can be bubbled through the gelling agent. Suitable gelling agents include, for example, a divalent cation such as alkali metal salt, alkaline earth metal salt or a transition metal salt that can ionically crosslink with the gelling agent. An inorganic salt, for example, a calcium, barium, zinc or magnesium salt can be used as a gelling agent. In embodiments, particularly those using an alginate gelling precursor, a suitable gelling agent is calcium chloride. The calcium cations have an affinity for carboxylic groups in the gelling precursor. The cations complex with carboxylic groups in the gelling precursor resulting in encapsulation of the base polymer in a matrix of gelling precursor.

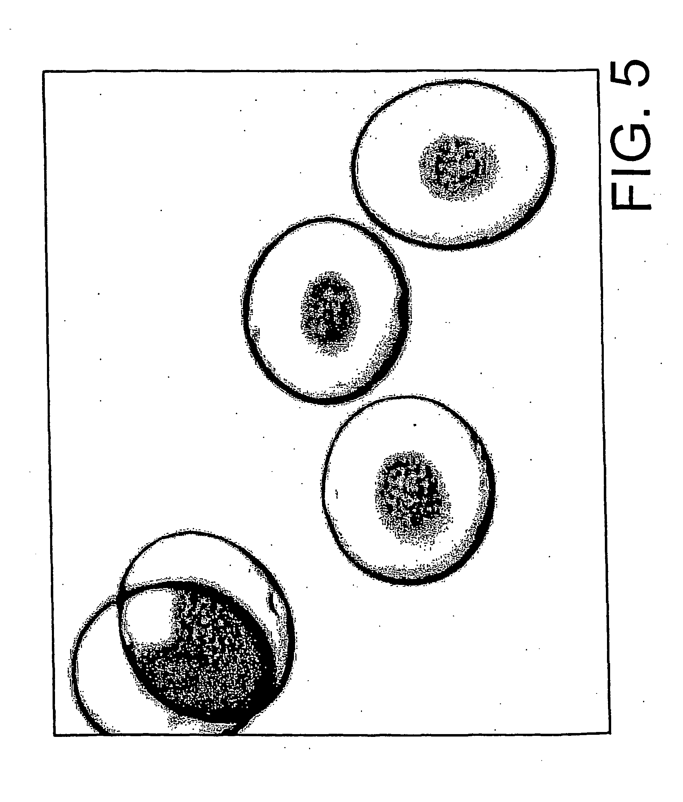

Referring to FIG. 5, a photo-image of the gelled particles, the gelling agent is in an amount selected in accordance with the desired properties of the particles. A pore structure in the center of the particle forms in the gelling stage. The concentration of the gelling agent can control void formation in the embolic particle, thereby controlling the porosity gradient in the embolic particle. Adding non-gelling ions, for example, sodium ions, to the gelling solution can limit the porosity gradient, resulting in a more uniform intermediate porosity throughout the particle. In this manner the thickness and pore profile of the metering region can be controlled. In embodiments, the gelling agent is, for example, 0.01-10 weight percent, 1-5 weight percent or 2 weight percent in deionized water.

Following drop stabilization, the gelling solution is decanted from the solid drops and the stabilized drops are transferred to the reactor vessel 330. In the reactor vessel 330, the stabilized drops are reacted to produce precursor particles. The reactor vessel includes an agent that chemically reacts with the base polymer, e.g. to cause crosslinking between polymer chains and/or within a polymer chain. The agent diffuses into the stabilized drops from the surface of the particle in a gradient, which, it is believed, provides more crosslinking near the surface of the stabilized drop compared to the body and center of the drop. Reaction is greatest at the surface of the drop, providing a stiff, abrasion resistant exterior. For polyvinyl alcohol, for example, the vessel 330 includes aldehydes, such as formaldehyde, glyoxal, benzaldehyde, aterephthalaldehyde, succinaldehydesuccinaldehyde, and glutaraldehyde for the acetalization of polyvinyl alcohol. The vessel 330 also includes an acid, for example, strong acids such as sulfuric acid, hydrochloric acid, nitric acid and weak acids such as acetic acid, formic acid and phosphoric acid. In embodiments, the reaction is primarily a 1,3 acetalization:

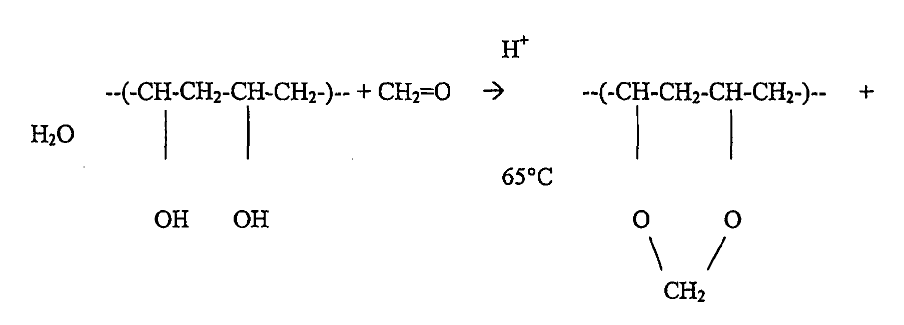

This intra-chain acetalization reaction can be carried out with relatively low probability of inter-chain crosslinking as described in John G. Pritchard "Poly(Vinyl Alcohol) Basic Properties And Uses (Polymer Monograph, vol. 4) (see p. 93-97), Gordon and Breach, Science Publishers Ltd., London, 1970. . Some OH groups along a polymer chain may remain unconverted since the reaction proceeds in a random fashion and there will be left over OH groups that do not react with adjacent groups.

Adjusting the amount of aldehyde and acid used, reaction time and reaction temperature can control the degree of acetalization. In embodiments, the reaction time is e.g., 5 minutes to 1 hour, 10 to 40 minutes or 20 minutes. The reaction temperature can be 25°C to 150°C or 75°C to 130°C or 65°C. The reactor vessel is placed in a water bath fitted with a orbital motion mixer. The crosslinked precursor particles are washed several times with deionized water to neutralize the particles and remove any residual acidic solution.

The precursor particles are transferred to the dissolution chamber 340 to remove the gelling precursor, e.g. by an ion exchange reaction. In embodiments, sodium alginate is removed by ion exchange with a solution of sodium hexa-metaphosphate (EM Science). The solution can include, for example, ethylenediaminetetracetic acid (EDTA), citric acid, other acids and phosphates. The concentration of the sodium hexa-metaphosphate can be, for example, 1-20 weight %, 1-10 weight % or 5 weight % in deionized water. Residual gelling precursor, for example, sodium alginate, can be determined by an assay for detection of uronic acids in, for example, alginates containing mannuronic and guluronic acid residues. Residual alginate, for example, may be present in the range of about 20-35% by weight prior to rinsing and in the range of about 0.01-0.5% or 0.1-0.3% or 0.18% in the particles after rinsing for 30 minutes in water at about 23°C.

The particles are filtered through filter 350 to remove residual debris. Particles of 500 to 700 microns are filtered through a sieve of 710 microns and then a sieve of 300 microns. Particles of 700 to 900 microns are filtered through a sieve of 1000 microns and then a sieve of 500 microns. Particles of 900 to 1200 microns are filtered through a sieve of 1180 microns and then a sieve of 710 microns.

The filtered particles are sterilized by a low temperature technique such as e-beam irradiation, and packaged, typically about 1 to 5 ml of particles in about 5 to 10 ml saline. In embodiments, electron beam irradiation can be used to pharmaceutically sterilize the particles to reduce bioburden. In e-beam sterilization, an electron beam is accelerated using magnetic and electric fields, and focused into a beam of energy. This resultant beam can be scanned by means of an electromagnet to produce a "curtain" of accelerated electrons. The accelerated electron beam penetrates the collection of embolic particles to confer upon them electrons which destroy bacteria and mold to sterilize and reduce the bioburden in the embolic particles. Electron beam sterilization can be carried out by sterilization vendors such as Titan Scan, Lima, Ohio.

The agent (e.g., therapeutic agent) can be incorporated in the particle at various stages. As discussed above, the agent may be added to the particle after particle formation. For example, the particle can be dried and rehydrated with the agent or a solution including the agent. Alternatively, the agent can be added during particle formation. For example, the agent can be mixed with PVA and alginate upstream of droplet formation or after droplet formation in the gelling vessel, reaction vessel, or dissolution chamber or in a separate step after any of these stages. The particles may also be used to deliver agent at the stabilized drop stage without crosslinking the base polymer or at the precursor particle stage with crosslinked base polymer with or without removing the gelling precursor or gelling agent. Alternatively, the agent can be provided only to the surface and/or metering region, e.g., by coating the particle, without including substantial amounts of agent in the interior portions of the particle, e.g., the reservoir region.

The particles can be coated to include a high concentration of agent (e.g., therapeutic agent) on their surface or loaded into the interior of the particle. The surface can release an initial dosage of agent after which the body of the particle can provide a burst release of agent, as discussed earlier. The agent on the surface can be the same as or different from the agent in the body of the particle. The agent on the surface can be applied by exposing the particle to a high concentration solution of the agent. The agent coated particle can include another coating over the surface the agent, e.g., a degradable polymer which erodes when the particle is administered or meters agent out flow from the surface, e.g., by providing a porous membrane. The coating can delay an initial burst of agent release. The coating can be applied by dipping or spraying the particle. The erodible polymer could be a polysaccharide, such as an alginate. Suitable material for alginate coatings are described in Edwards-Levy Biomaterials 1999, Nov 20 (21) 2069-84; J. Microencapsol. 1999 May-June 16(3); 291-301; and Takka et al. J. Microencapsol. 1999 May-June 16(3), 275-90. Other erodible coatings include water soluble polymers such as polyvinyl alcohol, e.g., that has not been cross-linked. Other coatings include biodegradable poly DL-lactide-poly ethylene glycol (PELA) discussed in Zhou et al. J. Control Release 2001 Jul. 10; 75;(1-2):27-36 or gelatin as discussed in Huang et al. Int. J. Pharm. 1995 May 10 182(1):93-100. Other coatings include hydrogels such as polyacrylic acid, haluronic acid, gelatin, or carboxymethyl cellulose. Other coatings include polyethylene glycols (PEG), chitosan, polyesters such as polycaprolactones, and poly(lactic-co-glycolic) acid (e.g., poly(d-lactic)coglycolic acid). Suitable coatings of these types are discussed in J. Control Release, vol. 78, 1-3, 17 Jan. 2002, pp. 15-24. The coatings can include agent or be substantially free of agent. The agent in the coating can be the same as or different from an agent on a surface layer of the particle and/or within the particle. A polymer coating, e.g. an erodible coating, can be applied to the particle surface in cases where a high concentration of agent has not been applied to the particle surface. The fluoroscopic visibility of the particle can be enhanced by incorporating a highly radiopaque material such as a metal, e.g. tantalum or platinum, into the polymer matrix of the particle or the coating.

The particles can be modified by chemical or physical modifications that affect attachment and/or release of the therapeutic agent, the visibility of the particles, or their shape. For example, the polymer of the particle can be modified by graft polymerization to, for example, provide a reactive side chain. A therapeutic agent is attached covalently or ionically to the reactive moiety of the graft polymer. A polymer that is grafted to the particle can be further polymerized to influence polymer chain length to create a molecular-level morphology or vary hydrophobicity. Suitable graft polymers include polymers with carboxylic acid, anhydride, or aceto-acetyl groups which can be grafted to, e.g. PVA side groups modified to provide acrylic acids. Graft polymerization is discussed in Biomaterials, 2002 Feb. 23 (3) 863-71 and "Polyvinyl Alcohol Developments," ed. C.A. Finch, John Whiley, 1992 (see especially sections 6.2.3 and 7.3.1). Suitable graft polymers also include peptides with cell binding domains. Examples are discussed in Hubbell, Biomacromolecules, 2002, vol. 3, 710-23. Species capable of cell membrane penetrations e.g. polyleucine oligomer can be attached to the particle to enhance cell attachment. Targeting ligands such as galactose can be introduced onto the surface of a particle. Galactose attachment onto polymers is discussed in Biotechnology Bioengineering, 2002 April 5 (78) 1-10. The grafted segment can be provided with reactive moieties such as amines, carboxylic acids or thiols to which therapeutic agent can be attached. The moieties can be used to modify the hydrophobic/hydrophilic and cationic/anionic nature of the particle surface. An example of a polymer that can be grafted is poly(vinyl alcohol)-graft-poly(lactic-co-glycolic acid) to produce brush-like branched polyesters for enhancing protein release, as discussed in Frauke-Pistel et al. J. Control Release 2001 May 18; 73(1):7-20. Particle charge and hydrophobicity can be modified by grafting. For example, a negatively charged hydrophilic backbone poly (2-sulfobutyl vinyl alcohol)-g-poly(lactide-co-glycolide) is described in Jung et al. J. Control Release 2000 Jul 3; 67(2-3):157-69.

The polymer of the particle can also be modified by, e.g. block copolymerization to provide reactive moieties for graft polymerization and/or for direct therapeutic agent attachment. The polymer can also be modified to provide reactive groups at specific sites. For example, hydroxyl groups of PVA can be modified to provide more reactive sites, such as e.g. amines, carboxylic acids, or thiols.

Release kinetics can also be modified by controlling crosslinking. Techniques for crosslinking PVA and controlling release kinetics are discussed in Kim et al. Pharmaceutical Research, vol. 9, No. 1 (1992); Cosmetic and Pharm. App. For Polymers, Aug. 1990 p.709-14; and Polymer Mater. Sci. Eng. (1990) vol. 63, p. 64-7. Crosslinking is also described in A.R. Bachtsi and C. Kiparissides Journal of Microencapsulation, 1995, vol. 12 part 1, p. 23-35; Tobata et al. J. Control Release vol. 50, part 1-3, p. 123-133; and Orenti et al., Arch. Pharm (Weinheim) 2000 Dec: 333 (12), 421-4 and Sappimath et al., J. Biomat. Sci. Polym. Ed. 2000j 11(i); 27-43.

The shape of the particles can be modified by physical deformation followed by crosslinking as described in U.S. Patent Application Serial No. 10/116,330, filed April 14, 2002 and entitled "Forming a Chemically Cross-Linked Particle of a Desired Shape and Diameter". The particles can be coated on or incorporated into other medical devices, such as implantable devices including stents, embolization coils, arterial filters, artificial heart valves, catheters, and balloons such as angioplasty balloons. Other medical delivery devices include wound dressings.

### Therapeutic Agents and Use

Therapeutic agents include materials that are biologically active to treat physiological conditions. The agent can be active in release from the particle to tissue or active as it resides in the particle and is exposed to tissue or body fluid in communication with the particle.

The term "therapeutic agent" includes one or more "therapeutic agents" or "drugs". The terms "therapeutic agents" and "drugs" are used interchangeably and include pharmaceutically active compounds, nucleic acids with and without carrier vectors such as lipids, compacting agents (such as histones), virus (such as adenovirus, adeno-associated virus, retrovirus, lentivirus and a-virus), polymers, hyaluronic acid, gene therapies, proteins, cells, stem cells and the like, or combinations thereof, with or without targeting sequences.

Specific examples of therapeutic agents include, for example, pharmaceutically active compounds, proteins, cells, stem cells, oligonucleotides, ribozymes, antisense oligonucleotides, DNA compacting agents, gene/vector systems (i.e., any vehicle that allows for the uptake and expression of nucleic acids), nucleic acids (including, for example, recombinant nucleic acids; naked DNA, cDNA, RNA; genomic DNA, cDNA or RNA in a noninfectious vector or in a viral vector and which further may have attached peptide targeting sequences; antisense nucleic acid (RNA or DNA); and DNA chimeras which include gene sequences and encoding for ferry proteins such as membrane translocating sequences ("MTS") and herpes simplex virus-1 ("VP22")), and viral, liposomes and cationic and anionic polymers and neutral polymers that are selected from a number of types depending on the desired application. Non-limiting examples of virus vectors or vectors derived from viral sources include adenoviral vectors, herpes simplex vectors, papilloma vectors, adeno-associated vectors, retroviral vectors, and the like. Non-limiting examples of biologically active solutes include anti-thrombogenic agents such as heparin, heparin derivatives, urokinase, and PPACK (dextrophenylalanine proline arginine chloromethylketone); antioxidants such as probucol and retinoic acid; angiogenic and anti-angiogenic agents and factors; agents blocking smooth muscle cell proliferation such as rapamycin, angiopeptin, and monoclonal antibodies capable of blocking smooth muscle cell proliferation; anti-inflammatory agents such as dexamethasone, prednisolone, corticosterone, budesonide, estrogen, sulfasalazine, acetyl salicylic acid, and mesalamine; calcium entry blockers such as verapamil, diltiazem and nifedipine; antineoplastic/antiproliferative/anti-mitotic agents such as paclitaxel, 5-fluorouracil, methotrexate, doxorubicin, daunorubicin, cyclosporine, cisplatin, vinblastine, vincristine, epothilones, endostatin, angiostatin and thymidine kinase inhibitors; antimicrobials such as iodine, triclosan, dephalosporins, aminoglycosides, and nitorfurantoin; anesthetic agents such as lidocaine, buplvacaine, and ropivacaine; nitrix oxide (NO) donors such as lisidomine, molsidomine, L-argine, NO-protein adducts, NO-carbohydrate adducts, polymeric or oligomeric NO adducts; anticoagulants such as D-Phe-Pro-Arg chloromethyl ketone, an RGD peptide-containing compound, heparine, antithrombin compounds, platelet receptor antagonists, antithrombin antibodies, anti-platelet receptor antibodies, enoxaparin, hirudin, Warafin sodium, Dicumarol, aspirin, prostaglandin inhibitors, platelet inhibitors and tick antiplatelet factors; vascular cell growth promoters such as growth factors, growth factor receptor antagonists, transcriptional activators, and translational promoters; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; survival genes which protect against cell death, such as anti-apoptotic Bcl-2 family factors and Akt kinase; and combinations thereof. Cells can be of human origin (autologous or allogenic) or from an animal source (xenogeneic), genetically engineered if desired to deliver proteins of interest at the injection site. The delivery mediated is formulated as needed to maintain cell function and viability. Any modifications are routinely made by one skilled in the art.

Useful polynucleotide sequences include DNA or RNA sequences having a therapeutic effect after being taken up by a cell. Examples of therapeutic polynucleotides include anti-sense DNA and RNA; DNA coding for an anti-sense RNA; or DNA coding for tRNA or rRNA to replace defective or deficient endogenous molecules. The polynucleotides can also code for therapeutic proteins or polypeptides. A polypeptide is understood to be any translation product of a polynucleotide regardless of size, and whether glycosylated or not. Therapeutic proteins and polypeptides include as primary example, those proteins or polypeptides that can compensate for defective or deficient species in an animal, or those that act through toxic effects to limit or remove harmful cells from the body. In addition, the polypeptides or proteins that can be injected, or whose DNA can be incorporated, include without limitation, angiogenic factors and other molecules competent to induce angiogenesis, including acidic and basic fibroblast growth factors, vascular endothelial growth factor, hif-1, epidermal growth factor, transforming growth factor α and β, platelet-derived endothelial growth factor, platelet-derived growth factor, tumor necrosis factor α, hepatocyte growth factor and insulin like growth factor; growth factors; cell cycle inhibitors including CDK inhibitors, anti-restenosis agents, including p15, p16, p18, p19, p21, p27, p53, p57, Rb, nFkB and E2F decoys, thymidine kinase ("TK") and combinations thereof and other agents useful for interfering with cell proliferation, including agents for treating malignancies; and combinations thereof. Examples of other angiogenesis inhibitors include Endostatin, Celecoxib, Herceptin, Iressa, Rosiglitazone, Taxol, Velcade, Zoledronic, Angiostatin, Bevacizumab, Avastin, Tetrahydrocortisol,Vitaxin, Arresten, Canstatin, Cleaved Antithrombin III, DBP-maf, PEDF, and Tumstatin. Still other useful factors, which can be provided as polypeptides or as DNA encoding these polypeptides, include monocyte chemoattractant protein ("MCP-1"), and the family of bone morphogenic proteins ("BMP's"). The known proteins include BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. These dimeric proteins can be provided as homodimers, heterodimers, or combinations thereof, alone, or together with other molecules. Alternatively, or in addition, molecules capable of inducing an upstream or downstream effect of a BMP can be provided. Such molecules include any of the "hedgehog" proteins, or the DNA's encoding them.

Therapeutic agents include one or more of the following therapeutic agents: cells, stem cells, virus, protein, drug, enzymes, or combinations thereof.

Organs and tissues that may be treated include any mammalian tissue or organ, whether injected *in vivo* or *ex vivo.* Non-limiting examples include heart, lung, brain, liver, skeletal muscle, smooth muscle, kidney, bladder, intestines, stomach, pancreas, ovary, prostate, eye, tumors, cartilage and bone.

Other examples of therapeutic agents include the following.

Immunologic species such as antigens captured from specific cell lines (e.g. cancerous) can be absorbed/adsorbed or attached to surface of a particle, which can then be injected at the targeted cell mass, tissue or organ, e.g. a cancer site, to begin an immunologic reaction/cascade/response. Examples include HuRx, and DCVax from Northwest BioTherapeutics Inc., Bothell, Washington. An antigen or genetically engineered molecule can also be used. For example, anti-EGF receptor antibodies, which help lengthen the time chemotherapy can be used as a treatment for colorectal cancer, can be used. Examples include Cetuximab from ImClone Systems, New York, New York. Antibodies or receptors, genetically engineered or not, can also be used. Monoclonal antibodies to cells of blood vessels interact in the angiogenesis cascade, which is important for the growth of tumors, can be used.

Proteins required for signaling pathways may be absorbed/adsorbed or attached to the surface of the particulate including antibodies, antigens, monoclonal antibodies, proteins found on cancer cells, proteins found on diseased cells in any system, proteins found on normal, nondiseased state cells in any system, or others. Signaling pathways of interest include pathways for cell regeneration, for cell death, for angiogenesis, for cell growth, for chronic heart failure, for cell differentiation or others. Suitable proteins include platelet derived growth factor BB as described in Bourke, 2002 Society for Biomaterials 28th Annual Meeting Transactions, page 144. Another particular therapeutic agent is vascular endothelial growth factor (VEGF) for enhancing endothelialization as described in J. Control Release, 2001, May 14, 14:72(1-3): 101-13.

Complete whole cells, pieces of cells, genetically engineered cells or cells made of components of more than one organism may be attached to the surface of the particulate. Treatment includes diabetes or any disease caused by the cells of that organ lacking in producing a specific hormone/protein/organic molecule, cancer or Alzheimer's disease or diseases caused by the cells producing an incorrect product that is not in their function to create.

Antimicrobial coatings could coat the surface of the PVA particulate to aid in lessening infection/immunologic response to the presence of these products in the body. Coatings include the use of zinc, silver, iodine, triclosan and/or ciprofloxacin in a resin/polymer such as polyurethane.

Antigrowth drugs for cancer treatment may be absorbed/adsorbed or attached to the surface of the particle. Examples include Herceptin and Gleevec from Genentech and Novartis respectively. Small molecule chemotherapy drugs are used for targeted cancer treatment. Examples include, Ethiodol, Doxorubicin, Cisplatin and Mitomycin-C.

Particular therapeutic agents for treatment of liver tumors include agents used in chemoembolization, such as carboplatin, cisplatin, doxorubicin, mytomycin and ethiodol, as discussed in Jean-Francois Geschwind, Dimitri Artemov et al., Journal of Vascular Interventional Radiology (2000) 11:1245-1255; Dheeraj Rajan, Michael Soulen et al, Journal of Vascular Interventional Radiology (2001) 12:187-193; and Leung, Goin and Sickle et al., Journal of Vascular Interventional Radiology (2001) 12:321-326. A particular tumor-toxic agent e.g. for liver treatment is paclitaxel, available from Bristol-Meyers Squib, New York, New York.

Particular therapeutic agents useful for treatment of uterine fibroid tumors include nonsteroidal anti-inflammatory medication, oral contraceptives, progestins, and gonadotrophin-releasing hormone agonists which may cause fibroid tumors to shrink as described in Levy et al., Journal of Women's Imaging 2(4):168-175, 2000. Other therapeutic agents for uterine fibroid shrinkage include lupron, as discussed in Lipman, Appl. Radiol. 29(7):15-20, 2000.

Therapeutic agents may also include agents which bind to specific biological environments. The agents could, for example be placed on the exterior of the particle to make the particle targetable. The particles can be used for oral or topical administration as well as percutaneous administration. The particles can be used in chemoembolization in which drug is injected to a site and the particles are used to embolize the vasculature. The particles can include the same agent, different agents, or no agent. The particles can be used in combination with hydrogel based aneurysm embolization systems as described in Cruise et al., 2002, Society for Biomaterials 28th Annual Meeting Transactions, page 203. Other applications include drug delivery for treatment of aneurisms, coronary artery disease, restenosis and benign prostatic hyperplasia, e.g., in combination with medical devices such as stents.

In some embodiments, the agent can be a radioactive species, such as a radioisotope or a radioactive molecule. In certain embodiments, a radioactive species can be a therapeutic agent (e.g., a tumor-toxic agent). In some embodiments, the agent is used as a radioactive label and/or to impart radiopacity to the particle. In some embodiments, an agent may act as a therapeutic agent, a radioactive label and/or a radiopacity enhancing agent.

Examples of radioisotopes include yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), holmium (¹⁶⁶Ho), samarium (¹⁵³Sm), iridium (¹⁹²Ir), rhodium (¹⁰⁵Rh), iodine (¹³¹I or ¹²⁵I), indium (¹¹¹In), technetium (⁹⁹Tc), phosphorus (³²P), sulfur (³⁵S), carbon (¹⁴C), tritium (³H), chromium (⁵¹Cr), chlorine (³⁶Cl), cobalt (⁵⁷Co or ⁵⁸Co), iron (⁵⁹Fe), selenium (⁷⁵Se), and/or gallium (⁶⁷Ga). In some embodiments, yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), holmium (¹¹⁶Ho), samarium (¹⁵³Sm), iridium (¹⁹²Ir), and/or rhodium (¹⁰⁵Rh) can be used as therapeutic agents. In certain embodiments, yttrium (⁹⁰Y), lutetium (¹⁷⁷Lu), actinium (²²⁵Ac), praseodymium, astatine (²¹¹At), rhenium (¹⁸⁶Re), bismuth (¹¹²Bi or ²¹³Bi), holmium (¹⁶⁶Ho), samarium (¹⁵³Sm), iridium (¹⁹²Ir), rhodium (¹⁰⁵Ph), iodine (¹³¹I or ¹²⁵I), indium (¹¹¹In), technetium (⁹⁹Tc), phosphorus (³²P), carbon (¹⁴C) , and/or tritium (³H) can be used as a radioactive label (e.g., for use in diagnostics).

Examples of radioactive molecules include antibodies containing one or more radioisotopes, for example, a radiolabeled antibody.

Radioisotopes that can be bound to antibodies include, for example, iodine (¹³¹I or ¹²⁵I), yttrium (⁹⁰Y), lutetium (¹⁷⁷Lu), actinium (²²⁵Ac), praseodymium, astatine (²¹¹At), rhenium (¹⁸⁶Re), bismuth (²¹²Bi or ²¹³Bi), indium (¹¹¹In), technetium (⁹⁹Tc), phosphorus (³²P) , rhodium (¹⁰⁵Rh), sulfur (³⁵S), carbon (¹⁴C), tritium (³H), chromium (⁵¹Cr), chlorine (³⁶Cl), cobalt (⁵⁷Co or ⁵⁸Co), iron (⁵⁹Fe), selenium (⁷⁵Se), and/or gallium (⁶⁷Ga). Examples of antibodies include monoclonal and polyclonal antibodies including RS7, Movl8, MN-14 IgG, CC49, COL-1, mAB A33, NP-4 F(ab')2 anti-CEA, anti-PSMA, ChL6, m-170, or antibodies to CD20, CD74 or CD52 antigens. Examples of radioisotope/antibody pairs include m-170 MAB with ⁹⁰Y. Examples of commercially available radioisotope/antibody pairs include Zevalin^{™} (IDEC pharmaceuticals, San Diego, CA) and Bexxar^{™} (Corixa corporation, Seattle, WA). Further examples of radioisotope/antibody pairs can be found in J. Nucl. Med. 2003, Apr: 44(4): 632-40.

Without wishing to be bound by theory, it is believed that using a radioisotope bound to an antibody as a therapeutic agent can allow for a particularly high degree of selectivity in treating a condition (e.g., a cancer condition) because, as known to those skilled in the art, the site of the condition (e.g., a cancerous lesion) can have antigens which recognize appropriate antibodies, which can allow the antibody to bind to the antigen and release the radioisotope. For example, an antibody that binds the prostate specific membrane antigen (PSMA) antibody, i.e., the anti-PSMA-antibody can be labeled as follows. The PSMA antibody is contacted with a chelating agent, e.g., 1,4,7,10-tetraazacyclododecane-N, N', N", N"'-tetraacetic acid (DOTA), to thereby produce a conjugated antibody. The conjugated antibody is radiolabeled with a radioisotope, e.g., indium (¹¹¹I), yttrium (⁹⁰Y), and lutetium (¹¹⁷Lu), to thereby produce a labeled anti-PSMA antibody. Examples of antibodies used in cancer treatment include Herceptin^{®} used to treat HER2-driven metastatic breast cancer, available from Genentech (San Francisco, CA) and Campath^{®} used to treat chronic B-cell lymphocytic leukemia, available from Berlex laboratories (Richmond, CA). Further examples of radioisotope/antibody pairs used for treating cancer can be found in J Nucl. Med. 2003 Mar; 44(3):465-74, Cancer Biother. Radiopharm. 2002 Dec; 17(6):681-7, J Nucl. Med. 2003 Jan; 44(1):77-84, and Am J Clin. Oncol. 2002 Dec; 25(6):541-6.

Antibodies can be radiolabeled by methods known in the art. Such methods are disclosed, for example, in Goldenberg et al., Clinical Cancer Research 5(10): 3079-3087, 1999. Additional methods can include methods to radiolabel proteins. Examples of commercially available reagents to radiolabel (e.g., iodinate) proteins include the Bolton & Hunter reagent for protein iodination (Amersham Biosciences, Piscataway, NJ 08855).

In general, a radioactive species (e.g., a radioisotope, a radioactive molecule) can be disposed in and/or on a particle. For example, a radioactive species can be absorbed/adsorbed or attached to the surface of a particle.

### Example 1

Particles are manufactured from an aqueous solution containing 8 weight % of polyvinyl alcohol, 99+% hydrolyzed, average M_{w} 89,000-120,000 (ALDRICH) and 2 weight% of gelling precursor, sodium alginate, PRONOVA UPLVG (FMC BioPolymer, Princeton, NJ) in deionized water and the mixture is heated to about 121°C. The solution has a viscosity of about 310 centipoise at room temperature and a viscosity of about 160 cps at 65°C. Using a syringe pump (Harvard Apparatus), the mixture is fed to drop generator (Nisco Engineering). Drops are directed into a gelling vessel containing 2 weight % of calcium chloride in deionized water and stirred with a stirring bar. The calcium chloride solution is decanted within about three minutes to avoid substantial leaching of the polyvinyl alcohol from the drops into the solution. The drops are added to the reaction vessel containing a solution of 4% by weight of formaldehyde (37 wt% in methanol) and 20% by weight sulfuric acid (95-98% concentrated). The reaction solution is stirred at 65°C for 20 minutes. Precursor particles are rinsed with deionized water (3 x 300 mL) to remove residual acidic solution. The sodium alginate is substantially removed by soaking the precursor particles in a solution of 5 weight % of sodium hexa-methaphosphate in deionized water for 0.5 hour. The solution is rinsed in deionized water to remove residual phosphate and alginate. The particles are filtered by sieving, as discussed above, placed in saline (USP 0.9% NaCl) and followed by irradiation sterilization.

Particles were produced at the nozzle diameters, nozzle frequencies, and flow rates (amplitude about 80% of maximum) described in Table I.

**TABLE 1**

| Bead | Nozzle | Frequency | Flow | Density | Sphericity | Suspendability |
|---|---|---|---|---|---|---|
| Size | Diameter | (kHz) | Rate | | | |
| (microns) | (microns) | | (mL/min) | (g/mL) | | (minutes) |
| 500-700 | 150 | 0.45 | 4 | - | 0.92 | 3 |
| 700-900 | 200 | 0.21 | 5 | 1.265 | 0.94 | 5 |
| 900-1200 | 300 | 0.22 | 10 | - | 0.95 | 6 |

Suspendability is measured at room temperature by mixing a solution of 2 ml of particles in 5 ml of saline and 5 ml of contrast solution (Omnipaque 300, Nycomed, Buckinghamshire, UK) and observing the time for about 50% of the particles to enter suspension, i.e. not sink to the bottom or float to the top of a container (about 10 ml, 25 mm diameter vial). Suspendability provides a practical measure of how long the particles will remain suspended in use. (Omnipaque is an aqueous solution of lohexol, N.N.-Bis (2,3-dihydroxypropyl)-T-[N-(2,3-dihydroxypropyl)-acetamide]-2,4,6-trilodo-isophthalamide; Omnipaque 300 contains 647 mg of iohexol equivalent to 300 mg of organic iodine per ml. The specific gravity of Omnipaque is 1.349 at 37°C and Omnipaque has an absolute viscosity of 11.8 cp at 20°C.) The particles remain in suspension for about 2-3 minutes.

Particle size uniformity and sphericity is measured using a Beckman Coulter RapidVUE Image Analyzer version 2.06 (Beckman Coulter, Miami, FL). Briefly, the RapidVUE takes an image of continuous-tone (gray-scale) form and converts it to a digital form through the process of sampling and quantization. The system software identifies and measures particles in an image in the form of a fiber, rod or sphere. Sphericity computation and other statistical definitions are in Appendix A, attached, which is a page from the RapidVUE operating manual.

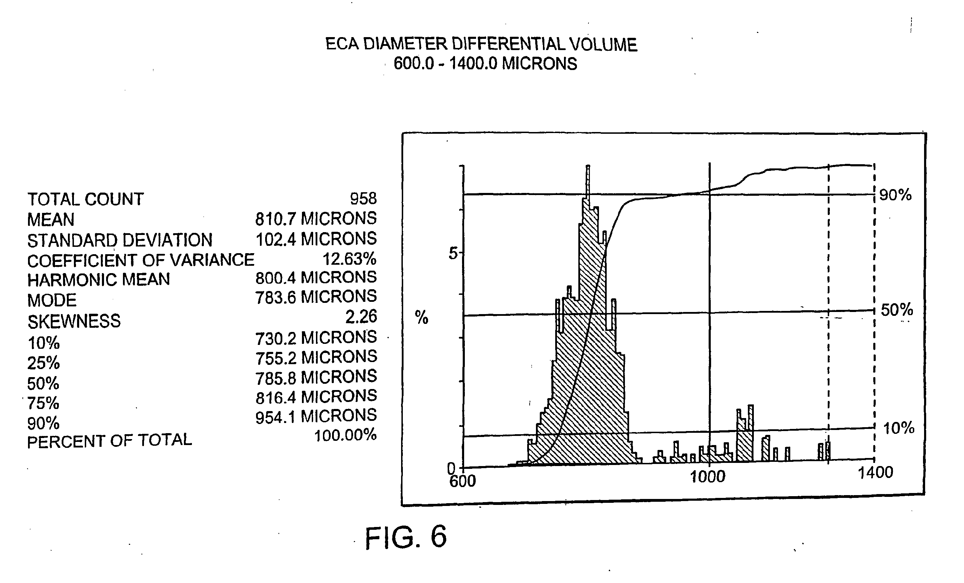

Referring to FIG. 6, particle size uniformity is illustrated for particles of about 700 - 900 microns. The x-axis is the particle diameter. The y-axis is the volume normalized percentage of particle at each particle size. The total volume of particles detected is computed and the volume of the particles at each diameter is divided by the total volume. The embolic particles have a distribution of particle sizes with a variance of less than about ± 15%.

The particles can be dried by lyophilization at -20 to 20°C and a pressure of about 75 mtorr for about 30 to 70 hours. The dried particles can be rehydrated by exposure to liquid. Exposure to contrast solution indicates that rehydration achieves entry of fluid throughout the particle.

### Example 2

Particles are made as described in example 1. Pharmaceutical grade Zevalin^{™} , available from IDEC pharmaceuticals (San Diego, CA) is incorporated into the particles as follows.

As acquired, the radioisotope is contained in a sterile glass v-vial. The glass v-vial is placed in an empty, first lead pot (for example, a 10 cm x 6 cm lead pot) behind a lead-shielded glass. Using a qualified dose calibrator, the activity of the radioisotope is calibrated to current time, and the activity is recorded, using a Decay Factor Chart that accompanies the radioisotope. The particles are drawn into a syringe and placed in a second lead pot. The first and second lead pots are placed behind a lead-shielded glass. A pre-calculated specific amount of particles, corresponding to a patient dose, is withdrawn from the second lead pot and placed in the glass v-vial containing the radioisotope. The radioactivity is recalibrated and corrected if necessary.

The particles are soaked in the solution of the radioisotope for about 15 minutes. The radioisotope is absorbed/adsorbed on/into the particles. The particles containing the radioisotope are placed in another sterile glass v-vial. The glass v-vial is transported to an implantation room, where implantation of particles into patients occurs.

For implantation, the particles loaded with the radioisotope are implanted into the patient using shielded glass syringes or shielded catheters.

Other embodiments are in the claims.

## Claims

1. A composition, comprising: a substantially spherical polymer particle comprising a cross-linked polymer matrix and having a diameter of about 1200 microns or less, wherein the particle contains an agent comprising a radioactive species, the particle includes a first region including pores having a first predominant pore size and a second region surrounding the first region and including pores having a second predominant pore size, and the first predominant pore size is larger than the second predominant pore size.

2. The composition of claim 1, wherein the agent comprises a therapeutic agent.

3. The composition of claim 1, wherein the radioactive species comprises a radioactive molecule.

4. The composition of claim 1, wherein the radioactive species comprises a radioisotope.

5. The composition of claim 4, wherein the radioisotope is selected from the group consisting of yttrium (⁹⁰Y) lutetium (¹⁷⁷Lu), actinium (¹¹⁵Ac), praseodymium, astatine (²¹¹At), rhenium (¹⁸⁶Re), bismuth (²¹²Bi or ²¹³Bi), holmium (¹⁶⁶Ho), samarium (¹⁵³Sm), iridium (¹⁹²Ir), rhodium (¹⁰⁵Rh), iodine (¹³¹I or ¹²⁵I), indium (¹¹¹In), technetium (⁹⁹Tc), phosphorus (³²P), sulfur (³⁵S), carbon (¹⁴C), tritium (³H), chromium (⁵¹Cr), chlorine (³⁶Cl), cobalt (⁵⁷Co or ⁵⁸Co), iron (⁵⁹Fe), selenium (⁷⁵Se), and gallium (⁶⁷Ga).

6. The composition of claim 4, wherein the radioisotope is bound to an antibody.

7. The composition of claim 6, wherein the antibody is selected from the group consisting of RS7, hRS7, MOv18, MN-14 IgG, CC49, COL-1, NP-4 F(ab') 2 anti-CEA, anti-PSMA, ChL6, m-170, antibodies to CD20, antibodies to CD74 and antibodies to CD52 antigens.

8. The composition of claim 6, wherein the antibody is a monoclonal antibody.

9. The composition of claim 8, wherein the monoclonal antibody is selected from the group consisting of mAB A33, m-170, antibodies to CD20, antibodies to CD74, and antibodies to CD52 antigens.

10. The composition of claim 1, wherein the polymer is selected from the group consisting of polyvinyl alcohol, polycaprolactone, polylactic acid and poly(lactic-co-glycolic) acid.

11. The composition of claim 1, wherein the polymer comprises polyvinyl alcohol.

12. The composition of claim 1, wherein the agent is in an interior of the particle.

13. The composition of claim 1, wherein the agent is on a surface region of the particle.

14. Use of a composition that comprises a substantially spherical polymer particle comprising a cross-linked polymer matrix and having a diameter of about 1200 microns or less for preparing a medicament wherein the particle contains an agent comprising a radioactive species, the particle including a first region including pores having a first predominant pore size and a second region surrounding the first region and including pores having a second predominant pore size, and the first predominant pore size being larger than the second predominant pore size

15. The use of claim 14, wherein the agent comprises a therapeutic agent.

16. The use of claim 14, wherein the radioactive species comprises a radioactive molecule.

17. The use of claim 14, wherein the radioactive species comprises a radioisotope.

18. The use of claim 14, wherein the composition is suitable to treat a cancer condition.

19. The use of claim 18, wherein the cancer condition is selected from the group of ovarian cancer, colorectal cancer, thyroid cancer, gastrointestinal cancer, breast cancer, prostate cancer and lung cancer. ,

20. The use of claim 14, wherein the radioisotope is bound to an antibody.

21. The use of claim 20, wherein the antibody is capable of binding to one or more antigens at a treatment site of the subject.

22. The use of claim 21, wherein the radioactive species is suitable to be released at the treatment site.

23. The use of claim 14, wherein the composition is suitable to be delivered by percutaneous injection.

24. The use of claim 14, wherein the composition is suitable to be delivered by a catheter.

25. A method of making a composition, the method comprising: disposing a radioactive species in a substantially spherical polymer particle comprising a cross-linked polymer matrix and having a diameter of about 1200 microns or less, the particle includes a first region including pores having a first predominant pore size and a second region surrounding the first region and including pores having a second predominant pore size, and the first predominant pore size is larger than the second predominant pore size.

26. The method of claim 25, wherein the radioactive species comprises a therapeutic agent.

27. The method of claim 25, wherein the radioactive species comprises a radioactive molecule.

28. The method of claim 25, wherein the radioactive species comprises a radioisotope.

29. The method of claim 25, further comprising disposing the radioactive species on a surface region of the particle.

30. A method of making a composition, the method comprising: disposing a radioactive species on a surface region of a substantially spherical polymer particle comprising a cross-linked polymer matrix and having a diameter of about 1200 microns or less, the particle includes a first region including pores having a first predominant pore size and a second region surrounding the first region and including pores having a second predominant pore size, and the first predominant pore size is larger than the second predominant pore size.

31. The method of claim 30, wherein the radioactive species comprises a therapeutic agent.

32. The method of claim 30, wherein the radioactive species comprises a radioactive molecule.

33. The method of claim 30, wherein the radioactive species comprises a radio-isotope.

## Patentansprüche

1. Zusammensetzung umfassend: ein im Wesentlichen kugelförmiges Polymerpartikel umfassend eine quervernetzte Polymermatrix und mit einem Durchmesser von ungefähr 1200 µm oder weniger, wobei das Partikel ein Mittel umfassend eine radioaktive Spezies enthält, wobei das Partikel einen ersten Bereich umfassend Poren mit einer ersten Hauptporengröße und einen zweiten Bereich umgebend den ersten Bereich und Poren mit einer zweiten Hauptporengröße umfasst, und wobei die erste Hauptporengröße größer ist als die zweite Hauptporengröße.

2. Zusammensetzung gemäß Anspruch 1, wobei das Mittel ein therapeutisches Mittel umfasst.

3. Zusammensetzung gemäß Anspruch 1, wobei die radioaktive Spezies ein radioaktives Molekül umfasst.

4. Zusammensetzung gemäß Anspruch 1, wobei die radioaktive Spezies ein Radioisotop umfasst.

5. Zusammensetzung gemäß Anspruch 4, wobei das Radioisotop ausgewählt ist aus der Gruppe bestehend aus Yttrium (⁹⁰Y), Lutetium (¹⁷⁷Lu), Actinium (²²⁵Ac), Praseodynium, Astatin (²¹¹At), Rhenium (¹⁸⁶Re), Bismuth (²¹²Bi oder ²¹³Bi), Holmium (¹⁶⁶Ho), Samarium (¹⁵³Sm), Iridium (¹⁹²Ir), Rhodium (¹⁰⁵Rh), Iod (¹³¹I oder ¹²⁵I), Indium (¹¹¹In), Technetium (⁹⁹Tc), Phosphor (³²P), Schwefel (³⁵S), Kohlenstoff (¹⁴C), Tritium (³H), Chrom (⁵¹Cr), Chlor (³⁶Cl), Kobalt (⁵⁷Co oder ⁵⁸Co), Eisen (⁵⁹Fe), Selen (⁷⁵Se) und Gallium (⁶⁷Ga).

6. Zusammensetzung gemäß Anspruch 4, wobei das Radioisotop an einen Antikörper gebunden ist.

7. Zusammensetzung gemäß Anspruch 6, wobei der Antikörper ausgewählt ist aus der Gruppe bestehend aus RS₇, hRS₇, MOv18, MN-14 IgG, CC49, COL-1, NP-4 F(ab')₂ anti-CEA, anti-PSMA, ChL6, m-170, Antikörper gegen CD₂₀, Antikörper gegen CD₇₄ und Antikörper gegen CD₅₂ Antigene.

8. Zusammensetzung gemäß Anspruch 6, wobei der Antikörper ein monoklonaler Antikörper ist.

9. Zusammensetzung gemäß Anspruch 8, wobei der monoklonale Antikörper ausgewählt ist aus der Gruppe bestehend aus mAB A₃₃, m-170, Antikörper gegen CD₂₀, Antikörper gegen CD₇₄ und Antikörper gegen CD₅₂ Antigene.

10. Zusammensetzung gemäß Anspruch 1, wobei das Polymer ausgewählt ist aus der Gruppe bestehend aus Polyvinylalkohol, Polycaprolacton, Polymilchsäure und Poly(Milchsäure-Co-Glykolsäure).

11. Zusammensetzung gemäß Anspruch 1, wobei das Polymer Polyvinylalkohol umfasst.

12. Zusammensetzung gemäß Anspruch 1, wobei das Mittel sich in einem Inneren des Partikels befindet.

13. Zusammensetzung gemäß Anspruch 1, wobei das Mittel sich auf einem Oberflächenbereich des Partikels befindet.

14. Verwendung einer Zusammensetzung, die ein im Wesentlichen kugelförmiges Polymerpartikel umfassend eine quervernetzte Polymermatrix und mit einem Durchmessen von ungefähr 1200 µm oder weniger umfasst zur Herstellung eines Medikamentes, wobei das Partikel ein Mittel umfassend eine radioaktive Spezies enthält, wobei das Partikel einen ersten Bereich umfassend Poren mit einer ersten Hauptporengröße und einen zweiten Bereich umfassend den ersten Bereich und enthaltend Poren mit einer zweiten Hauptporengröße umfasst, und wobei die erste Hauptporengröße größer ist als die zweite Hauptporengröße.

15. Verwendung gemäß Anspruch 14, wobei das Mittel ein therapeutisches Mittel umfasst.

16. Verwendung gemäß Anspruch 14, wobei die radioaktive Spezies ein radioaktives Molekül umfasst.

17. Verwendung gemäß Anspruch 14, wobei die radioaktive Spezies ein Radioisotop umfasst.

18. Verwendung gemäß Anspruch 14, wobei die Zusammensetzung zur Behandlung eines Krebszustandes ist.

19. Verwendung gemäß Anspruch 18, wobei der Krebszustand ausgewählt ist aus der Gruppe Gebärmutterhalskrebs, Kolorektalkrebs, Schilddrüsenkrebs, Magen-Darm-Krebs, Brustkrebs, Prostatakrebs und Lungenkrebs.

20. Verwendung gemäß Anspruch 14, wobei das Radioisotop an einen Antikörper gebunden ist.

21. Verwendung gemäß Anspruch 20, wobei der Antikörper geeignet ist zur Bindung an ein oder mehrere Antigene an einem Behandlungsort des Patienten.

22. Verwendung gemäß Anspruch 21, wobei die radioaktive Spezies geeignet ist, um an dem Behandlungsort freigesetzt zu werden.

23. Verwendung gemäß Anspruch 14, wobei die Zusammensetzung geeignet ist, um durch perkutane Injektion verabreicht zu werden.

24. Verwendung gemäß Anspruch 14, wobei die Zusammensetzung geeignet ist, um durch einen Katheter verabreicht zu werden.

25. Verfahren zur Herstellung einer Zusammensetzung, wobei das Verfahren umfasst: Einbringen einer radioaktiven Spezies in ein im Wesentlichen kugelförmiges Polymerpartikel, umfassend eine quervernetzte Polymermatrix und mit einem Durchmesser von ungefähr 1200 µm oder weniger, wobei das Partikel einen ersten Bereich umfassend Poren mit einer ersten Hauptporengröße und einen zweiten Bereich umfassend den ersten Bereich und enthaltend Poren mit einer zweiten Hauptporengröße umfasst, und wobei die erste Hauptporengröße größer ist als die zweite Hauptporengröße.

26. Verfahren gemäß Anspruch 25, wobei die radioaktive Spezies ein therapeutisches Mittel umfasst.

27. Verfahren gemäß Anspruch 25, wobei die radioaktive Spezies ein radioaktives Molekül umfasst.

28. Verfahren gemäß Anspruch 25, wobei die radioaktive Spezies ein Radioisotop umfasst.

29. Verfahren gemäß Anspruch 25 weiterhin umfassend das Einbringen der radioaktiven Spezies in einen Oberflächenbereich des Partikels.

30. Verfahren zum Herstellen einer Zusammensetzung, wobei das Verfahren umfasst: Einbringen einer radioaktiven Spezies in einen Oberflächenbereich eines im Wesentlichen kugelförmigen Polymerpartikels umfassend eine quervernetzte Polymermatrix und mit einem Durchmesser von ungefähr 1200 µm oder weniger, wobei das Partikel einen ersten Bereich umfassend Poren mit einer ersten Hauptporengröße und einen zweiten Bereich umfassend den ersten Bereich und enthaltend Poren mit einer zweiten Hauptporengröße umfasst, und wobei die erste Hauptporengröße größer ist als die zweite Hauptporengröße.

31. Verfahren gemäß Anspruch 30, wobei die radioaktive Spezies ein therapeutisches Mittel umfasst.

32. Verfahren gemäß Anspruch 30, wobei die radioaktive Spezies ein radioaktives Molekül umfasst.

33. Verfahren gemäß Anspruch 30, wobei die radioaktive Spezies ein Radioisotop umfasst.

## Revendications

1. Composition comprenant une particule polymère sensiblement sphérique, comprenant une matrice polymère réticulée et ayant un diamètre d'environ 1 200 µm ou moins, la particule contenant un agent comprenant une espèce radioactive, la particule comprenant une première région incluant des pores ayant une première grosseur de pore prédominante et une deuxième région entourant la première région et incluant des pores ayant une deuxième grosseur de pore prédominante, la première grosseur de pore prédominante étant plus grande que la deuxième grosseur de pore prédominante.

2. Composition selon la revendication 1, dans laquelle l'agent comprend un agent thérapeutique.

3. Composition selon la revendication 1, dans laquelle l'espèce radioactive comprend une molécule radioactive.

4. Composition selon la revendication 1, dans laquelle l'espèce radioactive comprend un radio-isotope.

5. Composition selon la revendication 4, dans laquelle le radio-isotope est choisi dans le groupe consistant en l'yttrium (⁹⁰Y) , le lutétium (¹⁷⁷Lu), l'actinium (²²⁵Ac), le praséodyme, l'astate (²¹¹At) , le rhénium (¹⁸⁶Re) , le bismuth (²¹²Bi ou ²¹³Bi) , le holmium (¹⁶⁶Ho), le samarium (¹⁵³Sm) , l'iridium (¹⁹²Ir) , le rhodium (¹⁰⁵Rh), l'iode (¹³¹I ou ¹²⁵I) , l'indium (¹¹¹In), le technétium (⁹⁹Tc) , le phosphore (³²P), le soufre (³⁵S), le carbone (¹⁹C), le tritium (³H), le chrome (⁵¹Cr), le chlore (³⁶Cl), le cobalt (⁵⁷Co ou ⁵⁸Co), le fer (^{S9}Fe), le sélénium (⁷⁵Se) et le gallium (⁶⁷Ga).

6. Composition selon la revendication 4, dans laquelle le radio-isotope est lié à un anticorps.

7. Composition selon la revendication 6, dans laquelle l'anticorps est choisi dans le groupe consistant en le RS7, le hRS7, le MOv18, l'IgG MN-14, le CC49, le COL-1, le NP-4 F(ab')2 anti-CEA, l'anti-PSMA, le ChL6, le m-170, les anticorps contre le CD20, les anticorps contre le CD74 et les anticorps contre les antigènes CD52.

8. Composition selon la revendication 6, dans laquelle l'anticorps est un anticorps monoclonal.

9. Composition selon la revendication 8, dans laquelle l'anticorps monoclonal est choisi dans le groupe consistant en l'anticorps monoclonal A33, le m-170, les anticorps contre le CD20, les anticorps contre le CD74 et les anticorps contre les antigènes CD52.

10. Composition selon la revendication 1, dans laquelle le polymère est choisi dans le groupe consistant en le poly(alcool vinylique), la polycaprolactone, le poly(acide lactique) et le poly(acide lactique-co-glycolique).

11. Composition selon la revendication 1, dans laquelle le polymère comprend du poly(alcool vinylique).

12. Composition selon la revendication 1, dans laquelle l'agent se trouve à l'intérieur de la particule.

13. Composition selon la revendication 1, dans laquelle l'agent se trouve sur une région de surface de la particule.

14. Utilisation d'une particule polymère sensiblement sphérique, comprenant une matrice polymère réticulée et ayant un diamètre d'environ 1 200 µm ou moins, pour préparer un médicament, la particule contenant un agent comprenant une espèce radioactive, la particule comprenant une première région incluant des pores ayant une première grosseur de pore prédominante et une deuxième région entourant la première région et incluant des pores ayant une deuxième grosseur de pore prédominante, la première grosseur de pore prédominante étant plus grande que la deuxième grosseur de pore prédominante.

15. Utilisation selon la revendication 14, pour laquelle l'agent comprend un agent thérapeutique.

16. Utilisation selon la revendication 14, pour laquelle l'espèce radioactive comprend une molécule radioactive.

17. Utilisation selon la revendication 14, pour laquelle l'espèce radioactive comprend un radio-isotope.

18. Utilisation selon la revendication 14, pour laquelle la composition convient au traitement d'un état cancéreux.

19. Utilisation selon la revendication 18, pour laquelle l'état cancéreux est choisi dans le groupe du cancer des ovaires, du cancer colorectal, du cancer de la thyroïde, du cancer gastro-intestinal, du cancer du sein, du cancer de la prostate et du cancer du poumon.

20. Utilisation selon la revendication 14, pour laquelle le radio-isotope est lié à un anticorps.

21. Utilisation selon la revendication 20, pour laquelle l'anticorps est capable de se lier à un ou plusieurs antigènes sur un site de traitement du sujet.

22. Utilisation selon la revendication 21, pour laquelle l'espèce radioactive est apte à être libérée au niveau du site de traitement.

23. Utilisation selon la revendication 14, pour laquelle la composition est apte à être administrée par injection percutanée.

24. Utilisation selon la revendication 14, pour laquelle la composition est apte à être administrée par un cathéter.

25. Procédé de fabrication d'une composition, le procédé comprenant la mise en place d'une espèce radioactive dans une particule polymère sensiblement sphérique comprenant une matrice polymère réticulée et ayant un diamètre d'environ 1 200 µm ou moins, la particule comprenant une première région incluant des pores ayant une première grosseur de pore prédominante et une deuxième région entourant la première région et incluant des pores ayant une deuxième grosseur de pore prédominante, et la première grosseur de pore prédominante étant plus grande que la deuxième grosseur de pore prédominante.

26. Procédé selon la revendication 25, dans lequel l'espèce radioactive comprend un agent thérapeutique.

27. Procédé selon la revendication 25, dans lequel l'espèce radioactive comprend une molécule radioactive.

28. Procédé selon la revendication 25, dans lequel l'espèce radioactive comprend un radio-isotope.

29. Procédé selon la revendication 25, qui comprend en outre la mise en place de l'espèce radioactive sur une région de surface de la particule.

30. Procédé de fabrication d'une composition, le procédé comprenant la mise en place d'une espèce radioactive d'une région de surface d'une particule polymère sensiblement sphérique comprenant une matrice polymère réticulée et ayant un diamètre d'environ 1 200 µm ou moins, la particule comprenant une première région incluant des pores ayant une première grosseur de pore prédominante et une deuxième région entourant la première région et incluant des pores ayant une deuxième grosseur de pore prédominante, et la première grosseur de pore prédominante étant plus grande que la deuxième grosseur de pore prédominante.

31. Procédé selon la revendication 30, dans lequel l'espèce radioactive comprend un agent thérapeutique.

32. Procédé selon la revendication 30, dans lequel l'espèce radioactive comprend une molécule radioactive.

33. Procédé selon la revendication 30, dans lequel l'espèce radioactive comprend un radio-isotope.
